# EUROPEAN PATENT SPECIFICATION

(11) **EP 0 350 714 B1**
(45) Date of publication and mention of the grant of the patent: **09.03.1994**
(21) Application number: 89111726.9
(22) Date of filing: 28.06.1989
(51) Int. Cl.: C12N 11/08, C07K 17/08, C12N 5/00, A61L 27/00

(54) **Tissue immobilization and cell culturing system and method for affixing biologically active moieties to a substrate**
Gewebe-Immobilisierungs- und Zellkultursystem und Verfahren zum Anbringen biologisch wirksamer Teile an einem Substrat
Système d'immobilisation de tissu et de culture de cellules et procédé pour attacher des moitiés biologiquement actives à un substrat

(30) Priority: 13.07.1988 US 218629
(43) Date of publication of application: 17.01.1990
(73) Proprietor: COLLABORATIVE BIOMEDICAL PRODUCTS INC., Bedford Massachusetts 01730 (US)
(72) Inventor: Chaturvedi, Nishith, Farmington, Connecticut 06032 (US); Picciano, Paul T., Canton, Connecticut 06019 (US)
(74) Representative: Hoeger, Stellrecht & Partner

(56) References cited:
- EP-A- 0 223 289
- EP-A- 0 243 818
- EP-A- 0 280 155
- US-A- 3 910 819
- CHEMICAL ABSTRACTS, vol. 109, 1988, p. 338, abstract no. 11759z, Columbus, Ohio, US; & JP-A-62 57 562 (MITSUBISHI RAYON CO. LTD.) 13-03-1987
- CHEMICAL ABSTRACTS, vol. 108, 1988, p. 54, abstract no. 23066p, Columbus, Ohio, US; & JP-A-62 115 064 (AGENCY OF INDUSTRIAL SCIENCES AND TECHNOLOGY) 26-05-1987
- CHEMICAL ABSTRACTS, vol. 111, 1989, p. 430, abstract no. 180769z, Columbus, Ohio, US; & JP-A-63 264 069 (IDEMITSU KOSAN CO., LTD.) 31-10-1988
- CHEMICAL ABSTRACTS, vol. 100, 1984, p. 270, abstract no. 153441c, Columbus, Ohio, US; & JP-A-58 206 960 (SUSUMU INDUSTRIAL CO., LTD. YOKOGAWA HOKUSHIN DENKI K.K.)

## Description

### Field of the Invention:

This invention relates to the use of a synthetic polymer, polyallylamine, for the immobilization of tissue sections, cells and tissue components for histological, immunohistochemical or other staining or detection systems. Staining or detection protocols frequently include numerous steps which can involve the use of acids, bases, detergents, hydrolytic agents or combinations of such agents. Heretofore, a significant percentage of tissue sections or other samples have been lost from glass slides at some step in the rather lengthy procedures used in diagnostic/analytical staining, resulting in frustration and delay. It has now been discovered that polyallylamine exhibits a high retention efficiency for tissue sections to glass despite numerous harsh treatments.

Polyallylamine, a commercially available synthetic polymer, has been found to be ideally suited as a cellular adhesion promoter because it has been found to effect the attachment of cells to virtually any substrate. Applications in vitro include research diagnostics, cell product harvest and cell metabolism research. In vivo applications include the growth of confluent cell monolayers on the surface of prostheses, such as for hip implants, trachael reconstruction and especially cardiovascular prostheses.

### Description of the Prior Art:

The harvest of cells from tissue for maintenance and propagation in vitro by tissue culture is a major tool in medical and biochemical research. Tissue culture is the technique or process of propagating and/or supporting the metabolism of tissues or cells derived from organisms (plant or animal) in a formulated nutritive environment. Once isolated by gentle tissue dissociation, cells are incubated in nutritive media capable of supporting life functions. With few exceptions, cells require attachment to a substrate in order to perform normal metabolic functions, grow and divide. In tissue, the substrate which provides the matrix for cell growth consists of collagen, laminin, and fibronectin. In vitro, the substrate is most often plastic, although glass and microporous cellulosic filters are sometimes used as substitutes. Examples of cell uses produced via tissue culture include: (1) the study of the metabolism of the cell, the metabolism of parasites (i.e., viruses, bacteria, etc.) within the cell, the interactive metabolism of different cell types (i.e., epithelial cells, fibroblasts, immuno-competent cells, thymocytes, platelets, etc.), the effect of exogenous factors on cellular metabolism, the genetic composition of cells (in vitro diagnostics); (2) the production of specific compounds, i.e., genes, proteins or other cellular components; and (3) the re-implantation of cells as for skin, corneal grafts, brain, vascular grafts, and in vitro fertilization.

In recent years, collagen, laminin, and fibronectin have been extracted and purified from animal tissues and marketed to cell and tissue culture researchers as cellular adhesion promoters. Synthetic poly-D-lysine and poly-L-lysine have also been sold for such purposes. Many other substances including polyethyleneimine, gelatin, casein glues and solvent-borne acrylic glues have also been used for tissue section attachment to glass slides.

More recently Waite and Tanzer (Science, 1981) identified some of nature's most powerful adhesives, bioadhesive polyphenolic proteins, secreted by marine mussels which set under water and routinely cope with the forces of surf and tides. Formulations containing such extracted and purified bioadhesive polyphenolic proteins are currently on the market (CELL-TAK® adhesive, BioPolymers, Inc., Farmington, CT).

With the exception of poly-D-lysine, poly-L-lysine and synthetic polycations such as polyethyleneimine, these factors must be extracted from biological sources. Secondly, most of these factors have a shelf life of approximately four weeks at-20°C once in solution. Moreover, the use of synthetic polymers is preferred in order to avoid the possible introduction of biologically derived, trace but hazardous contaminants.

Poly-D-lysine and poly-L-lysine, which can be synthesized to provide high molecular weight substances at a reasonable cost, have been found not very effective for cell attachment when compared to the extracted bioadhesive polyphenolic proteins.

It has now been discovered that polyallylamine is a very efficient cell immobilization agent. In fact, it out-performs the current market leader, poly-L-lysine, and is comparable to, yet significantly less expensive than, the bioadhesive polyphenolic protein formulations.

The need for cellular adhesion promoters is based upon the fact that in vitro substrates such as plastic or glass are biologically inert and often do not provide sufficient substrate adhesion for adequate cell or tissue attachment. Specific examples illustrative of poor attachment efficiency include primary cell isolates, cells seeded at low densities and cells seeded in continuous flow systems such as bio-reactors or hollow tube culture systems. In addition, certain substrates such as some microporous filters or Teflon® materials used for vascular grafts do not permit any cell attachment due to low surface energy.

Although adhesion promoters have assisted with attachment problems to a significant degree, certain inadequacies are still noteworthy. First, their mode of action is based on the fact that, although physiological, these factors are not truly adhesive; they only provide a physical support and trapping for cells. Second, they are not easily utilized on a variety of substrates other than those conventionally used for cell culture (e.g., polystyrene, nitrocellulose) nor can each be used with equal effectiveness for all cell types. Third, a recognizable potential health hazard arises with certain cellular adhesion promoters, including, for example, the extraction of fibronectin from human blood.

For in vivo applications, confluent cell monolayers are desired on prostheses, especially cardiovascular prostheses. Endothelial cells normally line the lumen of such vessels and actively prevent thrombosis, which is a major problem in the management of patients with cardiovascular disease. These cells also produce basement membrane material, the matrix for further wound healing. Currently, these prostheses are made of Teflon®, a substrate which does not promote cell attachment. No other known attachment factor mediates cell attachment to Teflon®.

The use of polyallylamine obviates these problems. It attaches well to a variety of substrates in the presence of water and does not fail in a continuously humid environment. Polyallylamine has been found to rapidly attach to both a variety of substrates and a variety of cells, cell components and tissue sections. It can be stored at 4°C for at least 10 months and at room temperature for at least 1 month without degradation or loss of function. Further, it is commercially available and can be secured in large quantities at an economically attractive price.

Accordingly, it is an object of the present invention to provide preparations useful as adhesive factors to promote or augment attachment efficiency, rate and/or strength of adhesion, growth and specialized function of cells to tissue culture or non-tissue culture materials and substrates including plastic, glass, metals, microporous filters (cellulosic, nylon, glass fiber, polyester, polycarbonate, and other synthetic and natural materials including other synthetic polymeric materials and products resulting from modifications made to the aforementioned synthetic polymeric materials) and synthetic or alloplastic materials that can be used in tissue or prosthetic graft procedures (e.g., mechanical heart and Teflon® related vascular grafting materials).

Another object of the present invention is to demonstrate effectiveness in the firm immobilization of histological specimens which routinely undergo harsh treatments during processing.

A further object of the present invention is to provide preparations useful as adhesive factors to promote or augment attachment efficiency, rate and/or strength of adhesion of other biologically active moieties such as proteins, DNA, hormones and antibiotics to a variety of substrates, some of which are mentioned above.

A still further object of the present invention is to provide the preparation or layering of substrates with polyallylamine and the assays employed to investigate the effectiveness of such layers on the above-mentioned parameters.

### SUMMARY OF THE INVENTION

The foregoing and other objects, advantages, and features are accomplished by the present invention which provides a method of affixing viable cells, tissues and other biologically active moieties such as proteins, DNA, hormones and antibiotics, to a substrate comprising:
(1) applying a film-forming polyallylamine composition comprising from about 0.01 to about 1.0 % by weight polyallylamine to a substrate;
(2) removing extraneous materials not firmly attached to said substrate; and
(3) applying viable cells, tissues or other biologically active moieties to said substrate, whereby said moieties become affixed to said substrate.

Concentrations of and formulations comprising polyallylamine can be altered according to substrate type or the attachment requirements of the particular cell, tissue or biologically active moiety employed. When affixing viable cells, the presence of a nutritive environment is required in order for the cells to perform their normal metabolic cell functions.

### DETAILED DESCRIPTION OF THE INVENTION

The growth and normal metabolism of eukaryotic cells requires attachment to a substrate with the cell layer essentially extended face-to-face on the substrate. Conventionally, cell culture utilizes plastic substrates and, to a lesser degree, glass and microporous filters or beads for cell attachment and propagation. More recently, physiological substrates (collagen, laminin, fibronectin) and synthetic poly-D-and poly-L-lysine have been utilized for these purposes coated on plastic to avoid problems inherent in cell culture at low seeding densities, using freshly isolated cells or on substrates less suitable for attachment (e.g., Teflon®). It has been discovered that polyallylamine surprisingly provides a suitable alternative because of its high binding affinity for both tissues and cells and a variety of substrates, biological and inert.

The ability of polyallylamine to strongly attach to a variety of substrates permits the attachment, maintenance and growth of cells to surfaces that heretofore posed problems either because of their composition, their application, or the type of cell requiring attachment.

Substrates that can be used include plastic, glass, and microporous filters (e.g., cellulosic, nylon, glass fiber, polyester, polycarbonate) for conventional cell culture research and/or cell product harvesting from bio-reactors used in batch cell culture or in genetic engineering; hollow fiber tubes and beads for cell product harvesting; and prosthetic vascular graft materials such as polytetrafluoroethylene (Teflon®) and related materials.

Most of these surfaces carry a net negative charge and therefore tend to tightly bind polycationic materials such as polyallylamine. Cells carry a net negative charge and, as a result, are slightly repelled from untreated surfaces while being attracted to the intermediary polyallylamine which has a net positive charge. Polyallylamine increases attachment efficiency, attachment rate and strength of attachment. This latter parameter is critical in applications involving cell product harvesting procedures or re-implantation of cells on vascular grafts which involve the passage of fluids over cell monolayers. Moreover, cells that attach poorly following isolation from tissue or due to cell type, and cells that do not normally attach, such as blood cells and suspension tissue culture cells (histiocytic lymphomas, platelets, white and red blood cells, etc.) could also be attached to substrates through this intermediate.

Furthermore, the surprising ability of polyallylamine to strongly attach to a variety of substrates permits the attachment of many other biologically active moieties, such as DNA, proteins, hormones and antibiotics.

The application of polyallylamine to substrates is generally performed as follows. Depending on the final concentration per square centimeter (cm²) desired, about 1.0 to about 100 µl of polyallyamine ranging from about 0.1 to about 10 µg per µl are evenly applied per cm² of substrate. Acidic film-forming solutions of polyallylamine can be formed into films by rapid evaporation upon placing the coated substrate within a laminar flow hood. Alternatively, the polyallylamine can be adsorbed in a neutral to basic solution by incubation for 20 minutes on the dish or slide followed by aspiration of the liquid. Under these conditions, an even film of polyallylamine remains on the surface. The resulting film can be rinsed with water or sterile tissue culture medium for removal of non-adsorbed extraneous moieties. The substrate can be used immediately or dried for storage. Cells or other biologically active moieties to be attached to the film are adjusted to desired concentrations and added to the substrate in serum-free or serum-containing medium or buffer. In the case of attachment of cells, at various timed intervals, the cells are evaluated for attachment, growth, or function, or treated according to prescribed objectives or experiments requiring the attached cells in tissues culture. In the case of attachment of tissue sections immediately after sectioning, the appropriate sections (paraffin, epoxy, cryo, etc.) are lifted to the slide, dried as appropriate and processed.

Conversely, when desirable, the polyallylamine can be affixed to the biologically active moieties and then, the resultant biologically active moities can be affixed to the substrate. The foregoing method, in greater detail, comprises the steps of:
(1) dispersing said biologically active moieties in a serum-free solution;
(2) admixing a sterile formulation comprising polyallylamine containing from about 0.01 to about 1.0 % by weight polyallylamine with said dispersion of biologically active moieties, thereby attaching said polyallylamine to said biologically active moieties;
(3) recovering the resulting biologically active moieties; and
(4) affixing said recovered biologically active moieties to said substrate.

The following examples further illustrate the present invention. These examples are presented for illustrative purposes only and are not illustrated to limit the spirit or scope of the invention. Unless otherwise stated, all percentages and parts are by weight.

### Example 1

In this and the following examples, CELL-TAK® adhesive, a formulation employing the extracted and purified bioadhesive polyphenolic protein described by Waite and Tanzer (Science, Vol. 212, pages 1038-1040, May 29, 1981) and commercially available from BioPolymers, Inc. of Farmington, CT, is employed. The Cell-Tak® adhesive is stored at 4° C. in 5% (V/V) acetic acid. Three mammalian cell types were used to compare attachment to Cell-Tak® adhesive versus attachment to uncoated tissue culture plasticware and plasticware coated with polyallylamine and poly-L-lysine attachment factors. Anchorage dependent baby hamster kidney cells (BHK-21; ATCC CCL 10) were grown in Basal Medium Eagle's (BME) containing 10% calf serum and 10% tryptose phosphate broth. Human histiocytic lymphoma cells (U-937; ATCC CRL 1593) were grown in RPM1 1640 medium containing 10% calf serum. Lymphocytic cells (P3X63-Ag8.653; ATCC CRL 1580) were grown similarly except with 20% heat inactivated fetal bovine serum. These latter two cell types are anchorage independent and are, therefore, manipulated in suspension cultures.

All adhesives were coated on tissue plasticware by a solution casting method. For all experiments, microliter-volumes of the 10 mg/ml solutions were spread and dried onto 35 mm, 10 cm² plastic dishes for a final density of 0.5 to 5 µg/cm². After air drying, the plates received one ethanol (95% v/v) and two distilled water rinses.

The attachment assays were designed to quantitate attached cells after 20 min. incubation periods at 37.5°C. BHK cells were trypsinized from stock plates, washed in fresh medium by centrifugation and suspended in fresh RPM1 1640 with 10% calf serum at a density of 2 x 10⁵ cells/ml. U-937 and P3X cells, which are grown in suspension, were washed by centrifugation and resuspended to similar densities. Suspensions were seeded onto untreated tissue culture dishes (control) and dishes treated with attachment proteins or synthetic polymers. At 20 min., triplicate experimental and control plates were chosen at random for quantitation of unattached cells. These were removed from the dishes after gentle agitation and counted on a hemacytometer. Data were calculated as percent of cells attached by subtracting the number of unattached cells harvested from dishes (average of three) from the total number of cells plated, dividing the result by the total number of cells plated, and multiplying the quotient by 100%.

Conditions for this assay were established such that suboptimal binding would be obtained for CELL-TAK® adhesive coated plates. This enabled the observation of any polymer coating that exceeded the capacity of the extracted product. Table I shows the range of efficiencies obtained for the various adhesives. The data indicate that polyallylamine and poly-L-lysine are efficient mediators of cell attachment and that polyallylamine can be used effectively at much lower concentrations than either CELL TAK® adhesive or poly-L-lysine.

The growth rate of mammalian cells in the presence of these adhesives was assayed to evaluate any potential adverse effects caused by any adhesive. Baby hamster kindey cell (BHK) stocks were grown to confluency in MEM plus 15% calf serum, trypsinized and washed several times by centrifugation on MEM. Suspensions (5 x 10⁴ cells/ml) were seeded into untreated 35 mm dishes (control) and dishes with CELL-TAK® and polyallylamine and poly-L-lysine (5 g/cm²) in MEM with 15% calf serum. At various time points during the incubation at 37.5°C. with 5% CO₂, triplicate plates were removed. The attached cells were then trypsinized from the surface, washed and counted in a hemacytometer.

The growth rates of BHK-21 cells are unaffected by all adhesives tested, when compared to uncoated controls. It is important to note that growth rate is neither enhanced nor retarded in the presence of any of these adhesives.

**Table I**

| Percent Cell Attachment | | | | | |
|---|---|---|---|---|---|
| Adhesive | BHK-21 15 µg | Cell Type P3X 25 µg | U-937 | | |
| | | | 10µg | 15 µg | 25 µg |
| CELL-TAK® adhesive | 83 | 99 | 0 | 15 | 89 |
| poly-L-lysine | 97 | 93 | 91 | 97 | 94 |
| polyallylamine | 98 | 92 | 97 | 98 | 98 |
| Plastic | 19 | 17 | 20 | 13 | 14 |

### Example 2

Sections of paraffin-embedded liver tissue were cut to 10 µm, and picked up on glass microscope slides coated with CELL-TAK® adhesive, polyallylamine or poly-L-lysine at a density of 5 µg per slide. Three slides containing two sections each were prepared for polyallylamine and CELL TAK® adhesive. Ten sections were immobilized for poly-l-lysine.

After drying for one hour on a warm table set at 45°C, the slides were dewaxed by two 5-minute washes in xylene, followed by 3 washes in 95% ETOH, one wash in 70% ETOH, and one 3-minute water wash. The slides were then subjected to the following sequential treatments:

| | TREATMENT | NUMBER OF SECTIONS REMAINING | | |
|---|---|---|---|---|
| | | Polyallylamine | Poly-L-lysine | CELL-TAK® Adhesive |
| 1. | 100 mM PBS, pH 8, 15 min | 6 | 10 | 6 |
| 2. | 10 mM PBS, pH 7, 0.3% H₂O₂, 0.25% Triton X-100, 2 hr. | 6 | 9 | 6 |
| 3. | Running Water, 15 min. | 6 | 9 | 6 |
| 4. | Trypsin/EDTA (.05%/.02%), 30 min, 37°C | 6 | 9 | 6 |
| 5. | Trypsin/EDTA (.05%/.02%), 30 min, 37°C | 6 | 9 | 6 |
| 6. | Running water, 15 min. | 6 | 5 | 6 |
| 7. | 0.1% SDS in water, 15.5 hr | 6 | 0 | 6 |
| 8. | Running water, 25 min. | 6 | - | 6 |
| 9. | Pepsin, (5,000 U/ml, 20 min, 37°C | 6 | - | 6 |
| 10. | Running water bath, 15 min | 6 | - | 6 |

Polyallylamine and CELL-TAK® adhesive performed similarly throughout all of these treatments. Poly-L-lysine immobilized sections, however, were all lost at or before treatment #7.

## Claims

1. Method for affixing biologically active moieties to a substrate comprising:
(1) applying a film-forming polyallylamine composition comprising from about 0.01 to about 1.0% by weight of polyallylamine to a substrate;
(2) removing extraneous materials not firmly attached to said substrate; and
(3) applying biologically active moieties to said substrate, whereby said biologically active moieties become affixed to said substrate.

2. Method as defined in Claim 1 wherein from about 1.0 to about 100 µl of said film-forming composition containing from about 0.1 to about 10 µg/µl of polyallylamine per cm² of substrate is applied to said substrate.

3. Method as defined in Claim 1 wherein said film-forming composition is acidic and the portion thereof not firmly attached to said substrate is evaporated from said substrate.

4. Method as defined in Claim 1 wherein said film-forming composition is neutral or basic and, after an incubation period, the portion thereof not firmly attached to said substrate can be removed by aspiration.

5. Method as defined in Claim 1 wherein said film-forming composition is sterile.

6. Method as defined in Claim 1 wherein said substrate is rinsed to remove extraneous materials with water or a buffered solution.

7. Method as defined in Claim 1 wherein said substrate is selected from the group consisting of synthetic polymeric materials, glass, metals, and microporous filters.

8. Method as defined in Claim 7 wherein said substrate is polytetrafluoroethylene.

9. Method as defined in Claim 1 wherein the biologically active moieties are selected from the group consisting of tissue sections, cells, cellular components, serum and other body fluid components.

10. Method of affixing viable cells to a substrate comprising:
(1) applying a film-forming polyallylamine composition comprising from about 0.01 to about 1.0% by weight of polyallylamine to a substrate;
(2) removing extraneous materials not firmly attached to said substrate;
(3) applying viable cells to said substrate, whereby said cells become affixed to said substrate and, in the presence of a nutritive environment, perform normal metalbolic functions.

11. Method as defined in Claim 10 wherein from about 1.0 to about 100 µl of said film-forming composition containing from about 0.1 to about 10 µg/ µl of polyallylamine per cm² of substrate is applied to said substrate.

12. Method as defined in Claim 10 wherein said film-forming composition is acidic and the portion thereof not firmly attached to said substrate is evaporated from said substrate.

13. Method as defined in Claim 10 wherein said film-forming composition is neutral or basic and, after an incubation period, the portion thereof not firmly attached to said substrate can be removed by aspiration.

14. Method as defined in Claim 10 wherein said film-forming composition is sterile.

15. Method as defined in Claim 10 wherein said substrate is rinsed to remove extraneous materials with sterile aqueous serum-free tissue culture medium.

16. Method as defined in Claim 10 wherein said substrate is rinsed to remove extraneous materials with water or buffered solution.

17. Method as defined in Claim 10 wherein the viable cells are applied to said substrate in a serum-containing medium.

18. Method as defined in Claim 10 wherein the viable cells are applied to said substrate in a serum-free medium.

19. Method as defined in Claim 10 wherein said substrate is an in vitro substrate.

20. Method as defined in Claim 19 wherein said substrate is selected from the group consisting of synthetic polymeric materials, glass, metals, and microporous filters.

21. Method as defined in Claim 20 wherein said substrate is polytetrafluoroethylene.

22. Method as defined in Claim 10 wherein said substrate is an in vivo substrate not including a human or animal body.

23. Method as defined in Claim 22 wherein the substrate is selected from the group consisting of colllagen, laminin, fibronectin, prosthetic grafting materials and polylysine.

24. Method as defined in Claim 23 wherein said substrate is polytetrafluoroethylene.

25. A cell culturing system comprising a substrate, a film comprising polyallylamine on said substrate, viable cells afixed to said film-containing substrate, and a nutritive medium contacting said cells, whereby said cells perform normal metabolic cell functions.

26. A cell culturing system as defined in Claim 25 wherein the substrate is an in vitro substrate.

27. A cell culturing system as defined in Claim 26 wherein the substrate is selected from the group consisting of synthetic polymeric materials, glass, metals, and microporous filters.

28. A cell culturing system as defined in Claim 27 wherein said substrate is polytetrafluoroethylene.

29. A cell culturing system as defined in Claim 25 wherein said substrate is an in vivo substrate not including a human or animal body.

30. A cell culturing system as defined in Claim 29 wherein the substrate is selected from the group consisting of collagen, laminin, fibronectin, prosthetic grafting materials and polylysine.

31. A cell culturing system as defined in Claim 30 wherein said substrate is polytetrafluoroethylene.

32. Method of affixing biologically active moieties to a substrate, comprising:
(1) dispersing said biologically active moieties in a serum free solution;
(2) admixing a formulation comprising polyallylamine comprising from about 0.01 to about 1.0 % by weight polyallylamine with said dispersion of biologically active moieties, thereby attaching said polyallylamine to said biologically active moieties;
(3) recovering the resulting biologically active moieties; and
(4) affixing said recovered biologically active moieties to said substrate.

## Patentansprüche

1. Verfahren zum Fixieren von biologisch aktiven Komponenten an einem Substrat, wobei das Verfahren umfaßt:
(1) Aufbringen einer dünne Schichten bildende Polyallylamin-Zusammensetzung, welche ungefähr 0,01 bis ungefähr 1,0 Gew. % Polyallylamin umfaßt, auf ein Substrat;
(2) Entfernen von fremden Materialien, welche nicht fest an dem Substrat befestigt sind; und
(3) Aufbringen biologisch aktiver Komponenten auf das Substrat, wodurch die biologisch aktiven Komponenten an dem Substrat fixiert werden.

2. Verfahren nach Anspruch 1, worin ungefähr 1,0 bis ungefähr 100 µl der dünne Schichten bildenden Zusammensetzung, welche ungefähr 0,1 bis ungefähr 10 µg/µl Polyallylamin enthält, pro cm² des Substrats auf das Substrat aufgetragen werden.

3. Verfahren nach Anspruch 1, worin die dünne Schichten bildende Zusammensetzung sauer ist und der Teil hiervon, welcher nicht fest an dem Substrat fixiert ist, von dem Substrat durch Abdampfen entfernt wird.

4. Verfahren nach Anspruch 1, worin die dünne Schichten bildende Zusammensetzung neutral oder basisch ist und worin nach einer Inkubationszeit der Teil davon, der nicht fest an dem Substrat befestigt ist, durch Absaugen entfernt wird.

5. Verfahren nach Anspruch 1, worin die dünne Schichten bildende Zusammensetzung steril ist.

6. Verfahren nach Anspruch 1, worin das Substrat gespült wird, um fremdes Material mit Wasser oder einer gepufferten Lösung zu entfernen.

7. Verfahren nach Anspruch 1, worin das Substrat ausgewählt ist aus der aus synthetischen Polymermaterialien, Glas, Metallen und Mikroporenfiltern bestehenden Gruppe.

8. Verfahren nach Anspruch 7, worin das Substrat Polytetrafluoroethylen ist.

9. Verfahren nach Anspruch 1, worin die biologisch aktiven Komponenten aus der aus Gewebeschnitten, Zellen, Zellkomponenten, Serum- und anderen Körperflüssigkeitskomponenten bestehenden Gruppe ausgewählt sind.

10. Verfahren zum Fixieren von lebenden Zellen an einem Substrat, wobei das Verfahren umfaßt:
(1) Auftragen einer dünne Schichten bildenden Polyallylamin-Zusammensetzung auf ein Substrat, welche ungefähr 0,01 bis 1,0 Gew. % Polyallylamin umfaßt;
(2) Entfernen von fremden Materialien, welche nicht fest an dem Substrat befestigt sind;
(3) Aufbringen von lebenden Zellen auf das Substrat, wodurch die Zellen an dem Substrat befestigt werden und in Gegenwart einer Nährumgebung normale Stoffwechselfunktionen ausführen.

11. Verfahren nach Anspruch 10, worin ungefähr 1,0 bis 100 µl der dünne Schichten bildenden Zusammensetzung, welche ungefähr 0,1 bis ungefähr 10 µg/µl Polyallylamin enthält, pro cm² des Substrats auf das Substrat aufgebracht wird.

12. Verfahren nach Anspruch 10, worin die dünne Schichten bildende Zusammensetzung sauer ist und der Teil davon, der nicht fest an dem Substrat befestigt ist, von dem Substrat durch Abdampfen entfernt wird.

13. Verfahren nach Anspruch 10, worin die dünne Schichten bildende Zusammensetzung neutral oder basisch ist und worin nach einer Inkubationszeit der Teil davon, der nicht fest an das Substrat fixiert ist, mittels Absaugen entfernt werden kann.

14. Verfahren nach Anspruch 10, worin die dünne Schichten bildende Zusammensetzung steril ist.

15. Verfahren nach Anspruch 10, worin das Substrat gespült wird, um fremde Materialien mit einem sterilen, wäßrigen, serumfreien Gewebe-Nährmedium zu entfernen.

16. Verfahren nach Anspruch 10, worin das Substrat zur Entfernung von fremden Materialien mit Wasser oder einer gepufferten Lösung gespült wird.

17. Verfahren nach Anspruch 10, worin die lebenden Zellen auf das Substrat in einem serum-enthaltenden Medium aufgebracht werden.

18. Verfahren nach Anspruch 10, worin die lebenden Zellen auf das Substrat in einem serumfreien Medium aufgebracht werden.

19. Verfahren nach Anspruch 10, worin das Substrat ein In-vitro-Substrat ist.

20. Verfahren nach Anspruch 19, worin das Substrat ausgewählt ist aus der aus synthetischen polymeren Materialien, Glas, Metallen und mikroporösen Filtern bestehenden Gruppe.

21. Verfahren nach Anspruch 20, worin das Substrat Polytetrafluoroethylen ist.

22. Verfahren nach Anspruch 10, worin das Substrat ein In-vivo-Substrat ist, ausgenommen ein menschlicher oder tierischer Körper.

23. Verfahren nach Anspruch 22, worin das Substrat ausgewählt ist aus der aus Collagen, Laminin, Fibronectin, prothetischen Implantatmaterialien und Polylysin bestehenden Gruppe.

24. Verfahren nach Anspruch 23, worin das Substrat Polytetrafluoroethylen ist.

25. Zellkultursystem, umfassend ein Substrat, eine dünne, Polyallylamin umfassende dünne Schicht auf dem Substrat, lebende Zellen, welche an dem die dünne Schicht enthaltenden Substrat befestigt sind und ein Nährmedium, welches in Kontakt mit den Zellen steht, wodurch die Zellen normale Stoffwechselzellfunktionen ausführen.

26. Zellkultursystem nach Anspruch 25, worin das Substrat ein In-vitro-Substrat ist.

27. Zellkultursystem nach Anspruch 26, worin das Substrat ausgewählt ist aus der aus synthetischen Polymermaterialien, Glas, Metallen und mikroporösen Filtern bestehenden Gruppe.

28. Zellkultursystem nach Anspruch 27, worin das Substrat Polytetrafluoroethylen ist.

29. Zellkultursystem nach Anspruch 25, worin das Substrat ein In-vivo-Substrat ist, nicht einschließend einen menschlichen oder tierischen Körper.

30. Zellkultursystem nach Anspruch 29, worin das Substrat ausgewählt ist aus der aus Collagen, Laminin, Fibronectin, prothetischen Implantatmaterialien und Polylysin bestehenden Gruppe.

31. Zellkultursystem nach Anspruch 30, worin das Substrat Polytetrafluoroethylen ist.

32. Verfahren zum Befestigen biologisch aktiver Komponenten an einem Substrat, wobei das Verfahren umfaßt:
(1) Dispergieren der biologisch aktiven Komponente in einer serumfreien Lösung;
(2) Mischen einer Polyallylamin umfassenden Formulierung, welche ungefähr 0,01 bis ungefähr 1,0 Gew. % Polyallylamin enthält, mit der Dispersion der biologisch aktiven Komponenten, wodurch das Polyallylamin an den biologisch aktiven Komponenten befestigt wird;
(3) Wiedergewinnen der sich ergebenden biologisch aktiven Komponenten; und
(4) Befestigen der wiedergewonnenen biologisch aktiven Komponenten an dem Substrat.

## Revendications

1. Méthode pour attacher des fragments biologiquement actifs à un substrat comprenant :
(1) l'application d'une composition de polyallylamine formant un film, comprenant d'environ 0,01 à environ 1,0 % en masse de polyallylamine, à un substrat;
(2) l'élimination des matériaux étrangers non solidement fixés audit substrat; et
(3) l'application des fragments biologiquement actifs audit substrat, par laquelle lesdits fragments biologiquement actifs s'attachent audit substrat.

2. Méthode comme définie dans la revendication 1, dans laquelle on applique audit substrat d'environ 1,0 à environ 100 µl de ladite composition formant un film contenant d'environ 0,1 à environ 10 µg/µl de polyallylamine par cm² de substrat.

3. Méthode comme définie dans la revendication 1, dans laquelle ladite composition formant un film est acide et dans laquelle la portion de ladite composition non solidement fixée audit substrat est évaporée dudit substrat.

4. Méthode comme définie dans la revendication 1, dans laquelle ladite composition formant un film est neutre ou basique, et dans laquelle, après une période d'incubation, la portion de ladite composition non solidement fixée audit substrat peut être éliminée par aspiration.

5. Méthode comme définie dans la revendication 1, dans laquelle ladite composition formant un film est stérile.

6. Méthode comme définie dans la revendication 1, dans laquelle ledit substrat est rincé à l'eau ou à l'aide d'une solution tamponnée pour éliminer les matériaux étrangers.

7. Méthode comme définie dans la revendication 1, dans laquelle ledit substrat est sélectionné dans le groupe comprenant des matériaux polymères svnthétiques, du verte, des métaux et des filtres microporeux.

8. Méthode comme définie dans la revendication 7, dans laquelle ledit substrat est du polytétrafluoroéthylène.

9. Méthode comme définie dans la revendication 1, dans laquelle les fragments biologiquement actifs sont choisis dans le groupe comprenant des coupes tissulaires, des cellules, des composants cellulaires, du sérum ou d'autres composants de fluides corporels.

10. Méthode pour attacher des fragments biologiquement actifs à un substrat comprenant :
(1) l'application d'une composition de polyallylamine formant un film, comprenant d'environ 0,01 à environ 1,0 % en masse de polyallylamine, à un substrat;
(2) l'élimination des matériaux étrangers non solidement fixés audit substrat; et
(3) l'application de cellules viables audit substrat, par laquelle lesdites cellules s'attachent audit substrat, et, en présence d'un environnement nutritif, exécutent des fonctions métaboliques normales.

11. Méthode comme définie dans la revendication 10, dans laquelle on applique audit substrat d'environ 1,0 à environ 100 µl de ladite composition formant un film contenant d' environ 0,1 à environ 10 µg/µl de polyallylamine par cm² de substrat.

12. Méthode comme définie dans la revendication 10, dans laquelle ladite composition formant un film est acide et dans laquelle la portion de ladite composition non solidement fixée audit substrat est évaporée dudit substrat.

13. Méthode comme définie dans la revendication 10, dans laquelle ladite composition formant un film est neutre ou basique, et dans laquelle, après une période d'incubation, la portion de ladite composition non solidement fixée audit substrat peut être éliminée par aspiration.

14. Méthode comme définie dans la revendication 10, dans laquelle ladite composition formant un film est stérile.

15. Méthode comme définie dans la revendication 10, dans laquelle ledit substrat est rincé à l'aide d'un milieu de culture tissulaire stérile, aqueux, exempt de sérum, pour éliminer les matériaux étrangers.

16. Méthode comme définie dans la revendication 10, dans laquelle ledit substrat est rincé à l'eau ou à l'aide d'une solution tamponnée pour éliminer les matériaux étrangers.

17. Méthode comme définie dans la revendication 10, dans laquelle les cellules viables sont appliquées audit substrat dans un milieu contenant du sérum.

18. Méthode comme définie dans la revendication 10, dans laquelle les cellules viables sont appliquées audit substrat dans un milieu exempt de sérum.

19. Méthode comme définie dans la revendication 10, dans laquelle ledit substrat est un substrat in vitro.

20. Méthode comme définie dans la revendication 19, dans laquelle ledit substrat est sélectionné dans le groupe comprenant des matériaux polymères synthétiques, du verte, des métaux et des filtres microporeux.

21. Méthode comme définie dans la revendication 20, dans laquelle ledit substrat est du polytétrafluoroéthylène.

22. Méthode comme définie dans la revendication 10, dans laquelle ledit substrat est un substrat in vivo n'incluant pas un corps humain ou animal.

23. Méthode comme définie dans la revendication 22, dans laquelle ledit substrat est sélectionné dans le groupe comprenant le collagène, la laminine, la fibronectine, les matériaux de greffe prosthétique, et la polylysine.

24. Méthode comme définie dans la revendication 23, dans laquelle ledit substrat est du polytétrafluoroéthylène.

25. Système de culture de cellules comprenant un substrat, un film comprenant de la polyallylamine sur ledit substrat, des cellules viables attachées audit substrat contenant un film, et un milieu nutritif au contact desdites cellules, par lequel lesdites cellules exécutent des fonctions cellulaires métaboliques normales.

26. Système de culture de cellules comme défini dans la revendication 25, dans lequel le substrat est un substrat in vitro.

27. Système de culture de cellules comme défini dans la revendication 26, dans lequel le substrat est sélectionné dans le groupe comprenant des matériaux polymères synthétiques, du verte, des métaux et des filtres microporeux.

28. Système de culture de cellules comme défini dans la revendication 27, dans lequel ledit substrat est du polytétrafluoroéthylène.

29. Système de culture de cellules comme défini dans la revendication 25, dans lequel ledit substrat est un substrat in vivo n'incluant pas un corps humain ou animal.

30. Système de culture de cellules comme défini dans la revendication 29, dans lequel ledit substrat est sélectionné dans le groupe comprenant le collagène, la laminine, la fibronectine, les matériaux de greffe prosthétique, et la polylysine.

31. Système de culture de cellules comme défini dans la revendication 30, dans lequel ledit substrat est du polytétrafluoroéthylène.

32. Méthode pour attacher des fragments biologiquement actifs à un substrat comprenant :
(1) la dispersion desdits fragments biologiquement actifs dans une solution exempte de sérum;
(2) l'addition d'une formulation comprenant de la polyallylamine comprenant d' environ 0,01 à environ 1,0 % en masse de polyallylamine avec ladite dispersion de fragments biologiquement actifs, par laquelle ladite polyallylamine se fixe auxdits fragments biologiquement actifs;
(3) la récupération des fragments biologiquement actifs résultants ; et
(4) l'attachement desdits fragments biologiquement actifs audit substrat.
